# EUROPÄISCHE PATENTSCHRIFT

(11) **EP 3 247 786 B1**
(45) Veröffentlichungstag und Bekanntmachung des Hinweises auf die Patenterteilung: **26.02.2020**
(21) Anmeldenummer: 16700726.9
(22) Anmeldetag: 15.01.2016
(51) Int. Cl.: C12M 3/00, C12M 1/26, C12M 1/42, C12M 1/33, C12M 1/00

(54) **VORRICHTUNG ZUR ELEKTRISCHEN DESINTEGRATION VON ZELLVERBÄNDEN, SOWIE ANLAGE UND VERWENDUNG DER VORRICHTUNG ZUR HERSTELLUNG VON FUTTERMITTELZWISCHENPRODUKTEN UND FUTTERMITTELPRODUKTEN**
DEVICE FOR THE ELECTRICAL DISINTEGRATION OF CELL STRUCTURES, AND INSTALLATION AND USE OF THE DEVICE FOR PRODUCING FEED INTERMEDIATES AND FEED PRODUCTS
DISPOSITIF POUR LA DÉSINTÉGRATION ÉLECTRIQUE D'AMAS CELLULAIRES, INSTALLATION ET UTILISATION DU DISPOSITIF POUR LA FABRICATION DE PRODUITS INTERMÉDIAIRES ALIMENTAIRES POUR ANIMAUX ET PRODUITS ALIMENTAIRES POUR ANIMAUX

(30) Priorität: 21.01.2015 DE 202015000482 U
(43) Veröffentlichungstag der Anmeldung: 29.11.2017
(73) Patentinhaber: Hugo Vogelsang Maschinenbau GmbH, 49632 Essen (DE)
(72) Erfinder: HOLLAH, Clemens, 49688 Lastrup (DE); KRAMPE, Paul, 49632 Essen/ Olbg. (DE); FASTENAU, Andreas, 49661 Cloppenburg (DE)
(74) Vertreter: Eisenführ Speiser
(86) Internationale Anmeldenummer: PCT/EP2016/050724
(87) Internationale Veröffentlichungsnummer: WO 2016/116357

(56) Entgegenhaltungen:
- EP-A1- 2 792 739
- WO-A1-84/00378
- WO-A2-2014/186670
- DE-A1- 3 538 194
- DE-C1- 19 913 514
- DE-U1-202011 004 177
- DE-U1-202011 107 866
- DE-U1-202013 001 124
- US-A1- 2010 317 053
- DATABASE WPI Week 198014 Thomson Scientific, London, GB; AN 1980-25104C & SU 679 196 A (VESELOV YU S) 15 August 1979 (1979-08-15)

## Beschreibung

Die vorliegende Erfindung betrifft eine Vorrichtung zur elektrischen Desintegration von Zellverbänden, mit einer Kammer mit einem Einlass zur Aufnahme von Zellverbände enthaltendem Material, einem Auslass zu dessen Abgabe, und einer sich zwischen dem Einlass und dem Auslass erstreckenden Förderstrecke, einer Elektrodeneinheit, welche einen Elektrodenkörper aufweist, der innerhalb der Kammer zumindest abschnittsweise entlang der Förderstrecke angeordnet ist, wobei die Kammer eine Wand aufweist, die abschnittsweise oder vollständig elektrisch leitfähig und von dem Elektrodenkörper elektrisch isoliert ist, und wobei die Elektrodeneinheit dazu angepasst ist, zwischen dem Elektrodenkörper und der Wand ein elektrisches Feld zur elektrischen Desintegration zu erzeugen.

Vorrichtungen der vorstehend bezeichneten Art, beispielsweise bekannt aus DE 20 2013 001 124.6 oder DE 20 2011 004 177 U1, werden in unterschiedlichen Bereichen eingesetzt, vornehmlich zur Behandlung von Zellverbänden und/oder Zellen enthaltendem Material. Ein bisheriges Einsatzgebiet war insbesondere das Gebiet der Biogasanlagen und Kläranlagen. Ziel ist es grundsätzlich, mittels der Desintegration der Zellverbände die Verarbeitbarkeit des Zellverbände enthaltendem Materials zu verbessern. Bei Biogasanlagen und Kläranlagen dient die Desintegration beispielsweise dazu, die Erzeugung von Biogas zu begünstigen.

Allgemein werden durch das sogenannte Cracken der Zellverbände chemische Reaktionen der Zellverbände beziehungsweise der die Zellen bildenden Moleküle begünstigt.

Unter dem Begriff Desintegration wird allgemein die Zerkleinerung und der Aufschluss von Zellen oder Zellverbänden unter Einwirkung äußerer Kräfte verstanden.

Die elektrische Desintegration beruht auf dem Funktionsprinzip, Zellverbände einem elektrischen Feld auszusetzen, welches zwischen zwei Elektroden anliegt. Infolge der Einwirkung des elektrischen Felds auf die Zellen und die Zellverbände kommt es an den Zellmembranen zu Ladungsverschiebungen. Bei den bekannten Anlagen zur elektrischen Desintegration wird hierbei sodann ausgenutzt, dass die Zellen und Zellverbände sich innerhalb der Kammer, in der das elektrische Feld anliegt, bewegen. Durch die Bewegung der Zellen und Zellverbände ändert sich in Bezug auf deren jeweilige Zellmembran lokal die sie beeinflussende Feldstärke. Durch diese andauernde Änderung wird die Zellmembran und/oder der Zellverband Schwerkräften oder Vibrationen ausgesetzt, was zu einer Destabilisierung führt. Bei ausreichend starker Anregung wird der Zellverband aufgelockert oder aufgelöst. Bei stärkerer Beeinflussung werden die Zellmembranen kollabiert. Letzteres ist unter dem Begriff der Elektroporation bekannt. Der Effekt dieser Desintegration ist jener, dass die Verfügbarkeit insbesondere von Nährstoffen aus den Zellverbänden erhöht wird. Man spricht in diesem Zusammenhang vom vorgenannten Aufschluss. Beispielsweise bei Getreide als Futtermittel für Tiere, etwa Ferkel, wirkt sich ein verbesserter Aufschluss vorteilhaft auf die Futterverwertung durch die Tiere aus.

Die Erhöhung der Verfügbarkeit von Nährstoffen in einem Material ist folglich nicht nur bei Biogasanlagen, sondern insbesondere auch in der Futtermittelindustrie von großer Bedeutung. Aus DE 199 13 514 C1 ist beispielsweise bekannt, einen Rohstoff, beispielsweise Getreideschrot, mittels thermischer und mechanischer Behandlung aufzuschließen, um darin enthaltenen Nährstoffe in einem herzustellenden Zwischenprodukt oder Futtermittelfertigprodukt leichter zugänglich zu machen. Ziel ist es, im Bereich der Futtermittelindustrie insbesondere die Verdaulichkeit, Schmackhaftigkeit und vor allen Dingen Verwertbarkeit des Futters zu erhöhen, damit mit weniger Futtermitteleinsatz stärkerer Gewichtszuwachs bei Tieren erzielt werden kann. Da die Tierzucht- und Fleischverarbeitende Branche in hohem Maße von Kostendruck geprägt ist, besteht ein Bedarf nach möglichst effizienter Futtermittelgewinnung, um in möglichst geringer Zeit und mit möglichst geringem Ressourcen-Einsatz hohe Wachstumsraten bei den das Futtermittel konsumierenden Tieren zu erreichen. Weiterhin könnte die Anzahl pathogener Keime im Futter auf ein Minimum reduziert werden.

SU 679196 A bezieht sich auf ein Gerät zum Koagulieren von Proteinen aus einem durch das Gerät geführten Material. Das Gerät umfasst ein Gehäuse mit einem Einlass und einem Auslass und einer sich in Längsrichtung im Gehäuse erstreckenden drehbaren Förderschnecke. Die Förderschnecke umfasst zwei Windungen, die mit unterschiedlichen Polaritäten beaufschlagt werden, wodurch ein elektrisches Feld zwischen den Windungen der Förderschnecke erzeugt wird.

Der Erfindung lag somit die Aufgabe zugrunde, eine Vorrichtung der eingangs genannten Art dahingehend zu verbessern, dass sie einen effizienzsteigernden Einsatz in der Herstellung von Futtermittelzwischenprodukten und fertigen Futtermittelprodukten erlaubt.

Die Erfindung löst die ihr zugrunde liegende Aufgabe bei einer Vorrichtung der eingangs genannten Art, indem in der Förderstrecke eine Förderschnecke zum Fördern des Materials antreibbar angeordnet ist, wobei der Elektrodenkörper in einem Hohlraum der Förderschnecke aufgenommen ist. Die Erfindung macht sich die Erkenntnis zunutze, dass die eingangs bezeichnete Vorrichtung nicht nur in Biogasanlagen, sondern auch zur elektrischen Desintegration von Zellverbänden in schüttfähigem Material, wie beispielsweise Getreideschrot, erfolgreich zum Einsatz gebracht werden kann. Anders als bei flüssigem Material wie Biomasse ist das Desintegrieren von Zellverbänden in einem grundsätzlich festen, schüttfähigen Material allerdings schwieriger. Die Erfindung verfolgt den Ansatz, mittels der Förderschnecke einen sehr kontrollierten, in der Fördergeschwindigkeit exakt justierbaren Transport des Zellverbände enthaltendem Materials durch die Kammer der Desintegrationsvorrichtung hindurch zu gewährleisten. Mittels der, vorzugsweise motorisch, antreibbaren Förderschnecke wird es gemäß der Erfindung möglich, die Verweilzeit des Zellverbände enthaltenen Materials in der Kammer zur elektrischen Desintegration exakt zu dosieren. Dadurch, dass die Förderschnecke den Elektrodenkörper der Elektrodeneinheit in einem Hohlraum in sich aufnimmt, wird ferner ein Transport des Zellverbände enthaltenen Materials gewährleistet, der durch die Elektrode selbst nicht behindert wird. Die Anordnung ist zudem platzsparend, sodass auf kleinster Stellfläche in industriellen Fertigungsanlagen lange Förderstrecken oder viele parallele Vorrichtungen zur elektrischen Desintegration aufgestellt werden können.

In einer bevorzugten Ausführungsform weist die Förderschnecke eine Hohlwelle auf, und der Elektrodenkörper ist koaxial zu der Förderschnecke in der Hohlwelle angeordnet.

Weiter vorzugsweise besteht die Förderschnecke teilweise oder vollständig aus einem elektrisch nicht leitfähigen Material. Dies hat den Vorteil, dass die Förderschnecke selbst keine oder jedenfalls nur eine verminderte Beeinträchtigung der Feldausbreitung in der Kammer zwischen Elektrode und Kammerwand bewirkt. Dies wiederum wirkt sich positiv auf die Effizienz der elektrischen Desintegration aus.

Das elektrisch nicht leitfähige Material ist in einer bevorzugten Ausgestaltung ein elektrisch nicht leitfähiges, vorzugsweise organisches, Polymer. Besonders bevorzugt ist dieses Polymer als Duromer, beispielsweise Polyurethan, Elastomer, beispielsweise Hartgummi oder als Plastomer, beispielsweise Polycarbonat, oder eine Kombination mehrerer Polymere ausgewählt. Bevorzugt werden ebenfalls Thermoplaste wie beispielsweise Polypropylen.

Ebenfalls bevorzugt wird es, wenn das elektrisch nicht leitfähige Material ein elektrisch nicht leitfähiger keramischer Werkstoff ist. Besonders bevorzugt wird als solcher ein Karbid-Werkstoff, beispielsweise Siliciumkarbid eingesetzt. Des Weiteren ist eine Kombination aus verschiedenen Werkstoffen, beispielsweise Keramik und Polymer, bevorzugt. Dies wird beispielsweise erreicht, indem auf ein Rohr aus einem ersten Werkstoff ein oder mehrere Schneckenflügel aus einem zweiten Werkstoff aufgebracht werden, etwa mittels Stoffschluss, Kraftschluss oder Formschluss, vorzugsweise etwa mittels Kleben und/oder Schrauben.

In einer weiteren bevorzugten Ausführungsform der Erfindung ist der Elektrodenkörper in die Hohlwelle eingegossen. Vorzugsweise ist der Elektrodenkörper epoxidharzvergossen.

Ebenfalls wird erfindungsgemäß bevorzugt, wenn der Elektrodenkörper in der Hohlwelle von einem Isolieröl umgeben ist. Die Hohlwelle wird beispielsweise mit einem Isolieröl, auch bezeichnet als Transformatorenöl, aufgefüllt. Das Isolieröl ist vorzugsweise ein, insbesondere hochraffiniertes, Mineralöl oder ein Silikonöl.

Die Erfindung betrifft neben einer Vorrichtung gemäß den vorstehend beschriebenen Ausführungsformen auch eine Anlage zur Herstellung eines Futtermittelzwischenproduktes, mit einem Einlass zum Zuführen von Zellverbände enthaltendem Material, und einem Auslass zur Abgabe des behandelten Materials als Zwischenprodukt. Die Erfindung löst die ihr zugrundeliegende Aufgabe bei einer solchen Anlage, indem die Anlage eine stromabwärts des Einlasses und stromaufwärts des Auslasses angeordnete, das aus Richtung des Einlasses kommende Material aufnehmende und in Richtung des Auslasses wieder abgebende Vorrichtung zur elektrischen Desintegration von Zellverbänden aufweist, die nach einer der vorstehend beschriebenen bevorzugten Ausführungsformen ausgebildet ist. Hinsichtlich der erfindungsgemäßen Vorteile und Wirkungen der Vorrichtung zur elektrischen Desintegration wird auf die obigen Ausführungen verwiesen.

In einer bevorzugten Weiterbildung der erfindungsgemäßen Anlage weist diese eine Befeuchtungseinrichtung zur Vorkonditionierung, vorzugsweise mittels Sattdampf, des zugeführten Materials auf. Vorzugsweise ist die Vorrichtung zur elektrischen Desintegration stromabwärts oder stromaufwärts der Befeuchtungseinrichtung angeordnet. Bei der Vorkonditionierung des Zellverbände enthaltendem Materials wird dem Zellverbände enthaltendem Material Wärme und Wasserdampf, vorzugsweise unter Vermeidung von Kondensat, zugeführt. Dies bietet den Vorteil, dass der spezifische Energiebedarf pro Kilogramm Material reduziert wird, beziehungsweise bei gleichem Energiebedarf die Durchsatzleistung erhöht werden kann. Wird die Vorrichtung zur elektrischen Desintegration stromaufwärts der Befeuchtungseinrichtung angeordnet, ist die Wasseraufnahme des Zellverbände enthaltendem Materials im Anschluss an das Durchlaufen der Vorrichtung zur elektrischen Desintegration erhöht. Ist die Vorrichtung zur elektrischen Desintegration stromabwärts der Befeuchtungseinrichtung angeordnet, sorgen die bereits erhöhte Temperatur und Feuchtigkeitsbeladung des Zellverbände enthaltendem Materials für eine höhere Desintegrationsrate.

Ein weiterer Vorteil der Vorkonditionierung des Zellverbände enthaltendem Materials mit Wärme und Dampf ist eine Reduzierung der mikrobiellen Belastung des Produktes und eine Erhöhung der Verdaubarkeit desselben. Sofern das Zwischenprodukt später noch weiter verarbeitet werden soll, beispielsweise in Pellets gepresst werden soll, wird die Festigkeit jener Pellets später potentiell erhöht, und damit der Abrieb der Pellets beim Fördern und Transportieren der Pellets vermindert.

Die erfindungsgemäße Anlage weist in einer weiteren bevorzugten Ausführungsform eine Behandlungseinrichtung zum thermischen und/oder mechanischen Aufschluss des zugeführten Materials auf, wobei die Vorrichtung zur elektrischen Desintegration vorzugsweise stromaufwärts oder stromabwärts der Behandlungseinrichtung angeordnet ist. Zum thermischen und mechanischen Aufschluss kann beispielsweise ein Extruder eingesetzt werden, welcher, beispielsweise unter Zufuhr von Wärme das in ihm geförderte Produkt umwälzt und hohen Scherbeanspruchungen aussetzt.

Eine bevorzugte Alternative zum thermisch und mechanischen Behandeln mittels Extrudern ist beispielsweise ein Expander zur Druckkonditionierung des Zellverbände enthaltendem Materials. Extruder und Expander haben gemein, dass mit ihnen das geförderte Produkt thermisch/mechanisch aufgeschlossen wird. Dies drückt sich in einer Strukturveränderung des Materials aus. Bei der Förderung von beispielsweise Getreideschrot äußert sich dies in einer Verkleisterung der Stärkekörner, einer höheren Fettverfügbarkeit, einem verminderten Keimgehalt, dem Abbau von verdauungshemmenden Substanzen, und verbesserten Verarbeitungseigenschaften des geförderten Materials.

Durch die Aufspaltung der Stärkekörner wird die Reaktion der Stärke in Stärkeabbauprodukte, wie beispielsweise Dextrine und Maltose ermöglicht. Ferner wird die Entfaltung von Proteinen ermöglicht.

Nach dem Verlassen des Extruders bzw. Expanders wird das erhaltene Zwischenprodukt vorzugsweise abgekühlt, bevor es zu einem weiteren Futtermittelprodukt, beispielsweise in einer Anlage nach dem nachfolgend beschriebenen Aspekt, weiterverarbeitet wird. Das abgekühlte Zwischenprodukt ist optional alternativ oder zusätzlich allerdings auch ein bevorzugtes vertriebsfähiges Produkt, welches kundenseitig mit Wasser aufgemischt werden muss und sodann verwendet werden kann.

Die Erfindung betrifft in einem weiteren Aspekt auch eine Anlage zur Herstellung eines pelletförmigen Futtermittelproduktes und die Anlage umfasst erfindungsgemäß die vorstehend beschriebene Anlage zur Herstellung eines Futtermittelzwischenproduktes nach einer der vorstehend beschriebenen Ausführungsformen, sowie eine Pelletiereinrichtung, die dazu eingerichtet ist, das ihr zugeführte Zwischenprodukt aus der Anlage zur Herstellung des Futtermittelzwischenproduktes aufzunehmen, in Pellets zu verarbeiten, insbesondere zu pressen, und als pelletförmiges Futtermittelprodukt abzugeben. Hinsichtlich der Vorteile dieser Anlage wird auf die vorstehenden Ausführungen zur erfindungsgemäßen Vorrichtung zu dessen Integration verwiesen.

Neben der Vorrichtung zur elektrischen Desintegration selbst und den sie einsetzenden Anlagen betrifft die Erfindung in einem weiteren Aspekt auch die Verwendung einer Vorrichtung zur elektrischen Desintegration in der Herstellung eines Futtermittelzwischenproduktes, wobei die Vorrichtung zur elektrischen Desintegration eine Kammer mit einem Einlass zur Aufnahme von Zellverbände enthaltenem Material, einem Auslass zu dessen Abgabe, und einer sich zwischen dem Einlass und dem Auslass erstreckenden Förderstrecke, sowie eine Elektrodeneinheit aufweist, welche einen Elektrodenkörper aufweist, der innerhalb der Kammer zumindest abschnittsweise entlang der Förderstrecke angeordnet ist, wobei die Kammer eine Wand aufweist, die abschnittsweise oder vollständig elektrisch leitfähig und von dem Elektrodenkörper elektrisch isoliert ist, und die Elektrodeneinheit dazu angepasst ist, zwischen dem Elektrodenkörper und der Wand ein elektrisches Feld zur elektrischen Desintegration zu erzeugen. Insbesondere betrifft die Erfindung eine solche Verwendung der vorbezeichneten Art, bei der in der Förderstrecke der Vorrichtung zur elektrischen Desintegration eine Förderschnecke zum Fördern des Materials antreibbar angeordnet ist, wobei der Elektrodenkörper in einem Hohlraum der Förderschnecke aufgenommen ist.

In bevorzugten Weiterbildungen der erfindungsgemäßen Verwendung ist die Vorrichtung zur elektrischen Desintegration nach einer der hierin beschriebenen vorstehenden Ausführungsformen ausgebildet.

In einem weiteren Aspekt betrifft die Erfindung auch die Verwendung einer Vorrichtung zur elektrischen Desintegration der Herstellung eines Futtermittelproduktes, wobei die Vorrichtung zur elektrischen Desintegration vorzugsweise ebenso ausgebildet ist, wie vorstehend unter Bezugnahme auf die Verwendung zur Herstellung eines Futtermittelzwischenproduktes bereits erläutert wurde.

Die erfindungsgemäße Vorrichtung wurde im Vorstehenden bezüglich ihrer bevorzugten Eignung zur elektrischen Desintegration von Zellverbände enthaltendem Material beschrieben. Neben ihrer Eignung und Verwendung zu jenem Zweck ist allerdings die Vorrichtung auch im besonderen Maße dazu geeignet, die Oberflächenladung von Partikeln zu modifizieren. Somit betrifft die Erfindung in einem weiteren Aspekt eine Vorrichtung zur elektrischen Modifikation der Oberflächenladung von Partikeln, mit einer Kammer mit einem Materialeinlass, einem Materialauslass, und einer sich zwischen dem Materialeinlass und dem Materialauslass erstreckenden Förderstrecke, einer Elektrodeneinheit, welche einen Elektrodenkörper aufweist, der innerhalb der Kammer zumindest abschnittsweise entlang der Förderstrecke angeordnet ist, wobei die Kammer eine Wand aufweist, die abschnittsweise oder vollständig elektrisch leitfähig und von dem Elektrodenkörper elektrisch isoliert ist, und wobei die Elektrodeneinheit dazu angepasst ist, zwischen dem Elektrodenkörper und der Wand ein elektrisches Feld zur Modifikation der Oberflächenladung zu erzeugen, wobei in der Förderstrecke eine Förderschnecke zum Fördern des Materials antreibbar angeordnet ist, wobei der Elektrodenkörper in einem Hohlraum der Förderschnecke aufgenommen ist.

Die Vorrichtung gleicht strukturell somit der vorstehend beschriebenen Vorrichtung zur elektrischen Desintegration von Zellverbänden enthaltendem Material, mit der Ausnahme, dass im Betrieb in die Kammer kein Zellverbände enthaltendes Material, sondern ein partikelhaltiges Material eingeführt und aus ihr wieder ausgeführt wird. Erfindungsgemäß werden unter Partikeln disperse Materialien verstanden, die sich vom umgebauten, kontinuierlichen Medium durch eine Phasengrenzfläche unterscheiden. Dies kann beispielsweise die festen oder flüssigen Bestandteile von Aerosolen, die festen Bestandteile von Suspensionen, die Teilchen von Pulvern, oder auch Flüssigkeitstropfen in Emulsionen betreffen. Durch Modifikation der Oberflächenladung der Partikel lassen sich Stoffe, die sich in unbehandeltem Zustand statisch abstoßen, nach dem Durchlaufen der Vorrichtung zur elektrischen Modifikation der Oberflächenladung besser verarbeiten, beispielsweise in Mischapparaturen. Anwendung finden kann dies beispielsweise bei Öltröpfchen enthaltenem Fluid, bei Lacken, oder Suspensionen von Stärkepulvern.

Die Vorrichtung zur elektrischen Modifikation der Oberflächenladung von Partikel macht sich die gleichen Erkenntnisse und Vorteile wie die weiter oben beschriebene Vorrichtung zur elektrischen Desintegration zu Nutze und verfügt über die gleichen bevorzugten Ausführungsformen, weswegen insoweit auf die vorstehenden Ausführungen verwiesen wird.

Somit betrifft die Erfindung neben einer Verwendung der beschriebenen Vorrichtung zur elektrischen Desintegration von Zellverbänden enthaltenem Material ebenso die Verwendung der Vorrichtung zur elektrischen Modifikation der Oberflächenladung von Partikeln, wobei die Vorrichtung nach einer der hier beschriebenen bevorzugten Ausführungsformen ausgebildet ist.

Im Folgenden wird die Erfindung anhand bevorzugter Ausführungsbeispiele unter Bezugnahme auf die beigefügten Figuren näher beschrieben. Hierbei zeigen
- Figur 1: eine Vorrichtung zur elektrischen Desintegration gemäß einem bevorzugten Ausführungsbeispiel, und
- Figur 2: eine Anlage zur Herstellung eines Futtermittelprodukts in ihrem schematischen Aufbau gemäß einem bevorzugten Ausführungsbeispiel.

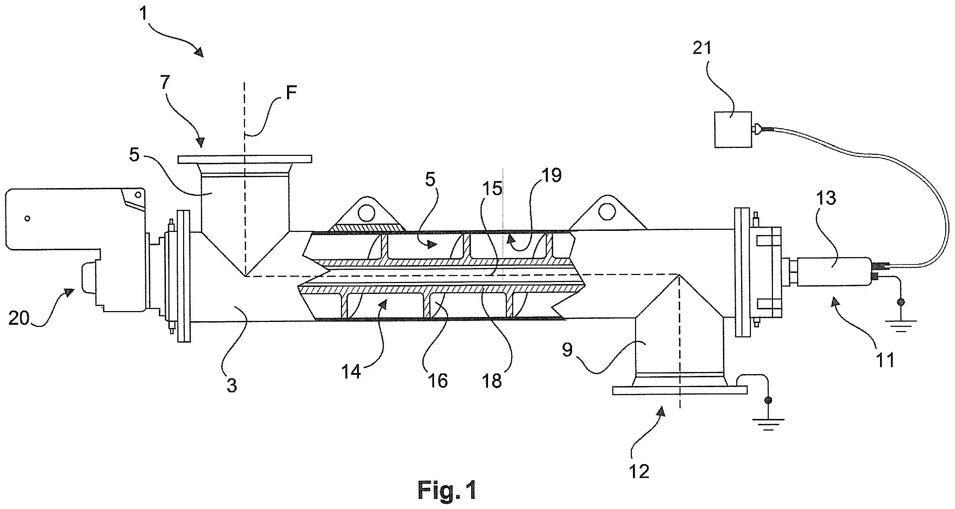

Der schematische Aufbau der erfindungsgemäßen Vorrichtung zur elektrischen Desintegration ist in teilweiser Schnittdarstellung in Figur 1 gezeigt. Die Vorrichtung 1 zur elektrischen Desintegration weist einen Grundkörper 3 auf. Der Grundkörper 3 ist geerdet. An dem Grundkörper 3 ist ein erster Stutzen 5 angeordnet, der einen Einlass 7 zum Einführen von Zellverbände oder Partikel enthaltendem Material in die Vorrichtung 1 aufweist. Ferner ist an dem Grundkörper 3 ein zweiter Stutzen 9 angeordnet, der einen Auslass 12 zur Abfuhr von dem zuvor eingeführten Material aufweist. Zwischen Einlass 7 und Auslass 12 erstreckt sich eine Förderstrecke, gekennzeichnet durch Strichellinie F. In einer alternativen, hier nicht gezeigten Ausgestaltung werden die Elektrodeneinheit und der Antrieb bevorzugt in umgekehrter Reihenfolge angeordnet.

Im Inneren des Grundkörpers 3 ist eine Kammer 5 ausgebildet, durch welche das Partikel oder Zellverbände enthaltende Material hindurchgefördert wird. Die Kammer weist eine Förderschnecke 14 auf. Die Förderschnecke 14 weist mehrere Förderwendel 16 auf, die möglichst spaltfrei an einer Wand 19 der Kammer 5 anliegen, um eine möglichst rückflussarme Förderung des Partikel oder Zellverbände enthaltenden Materials zu gewährleisten. Die Förderwendel 16 sind auf einer Hohlwelle 18 angeordnet. Die Förderschnecke 14 ist vorzugsweise mittels eines elektromotorischen Antriebs 20 antreibbar bzw. steuerbar. Alternative Antriebsarten sind vorzugsweise hydraulische Antriebe, Gasmotoren, oder mechanische Antriebe (beispielsweise Riementriebe).

Die Vorrichtung 1 weist ferner eine Elektrodeneinheit 11 auf, welche einen Elektrodenkopf 13 und einen Elektrodenkörper 15 umfasst, wobei der Elektrodenkörper 15 innerhalb der Kammer 5 entlang der Förderstrecke F angeordnet ist. Die Elektrodeneinheit 11 ist geerdet. Der Elektrodenkörper 15 ist innerhalb der Hohlwelle 18 aufgenommen. Die Elektrodeneinheit wird durch eine Steuereinheit 21 angesteuert. Die Steuereinheit ist vorzugsweise prinzipiell wie beschrieben in DE 20 2013 009 847 ausgebildet.

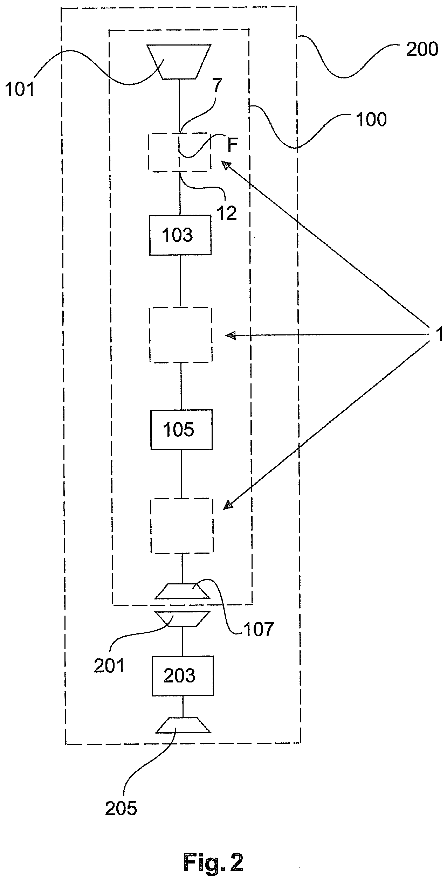

Im Betrieb wird mittels der Elektrodeneinheit 11 innerhalb der Kammer 5 ein elektrisches Feld zwischen dem Elektrodenkörper 15 und der Wand 19 der Kammer erzeugt, welches auf das entlang der Förderstrecke F geförderte Zellverbände enthaltende Material bzw. die entlang der Förderstrecke F geförderten Partikel einwirkt und bei ausreichender Feldstärke bei dem Zellverbände enthaltenden Material zu einer Elektroporation, bzw. elektrischen Desintegration von Zellverbänden bzw. bei dem Partikel enthaltenden Material zu einer elektrischen Modifikation der Oberflächenladung, insbesondere einer Entladung, führt. Es ist ebenfalls möglich, dass bei dem die Zellverbände enthaltendem Material in ähnlicher Weise wie bei den Partikeln die Oberflächenladung geändert wird, wodurch es zu einem verbesserten Aufschluss kommt. Dieses kommt bei Zellverbände enthaltendem Material vor allem dann in Betracht, wenn es zwischen einem Einlass 101 und einer Befeuchtungseinrichtung 103 einer Anlage 200 zur Herstellung von Futtermittelzwischenprodukten oder Futtermittelprodukten (vgl. Figur 2) angeordnet ist. Durch diese Anordnung kann beispielsweise die Wasseraufnahmerate (Benetzung der Partikel) gesteigert werden, und es kommt dann beim Extrudieren/Expandieren zu einer höheren spez. Energiefreisetzung.

Über die Drehzahl der Förderschnecke 14 kann der Bediener die Verweilzeit des Partikel bzw. Zellverbände enthaltenden Materials (beispielsweise Getreideschrot, vorkonditioniertes Getreideschrot oder bereits thermisch und mechanisch aufgeschlossenes Getreideschrot) exakt vorgeben. Auf diese Weise wird vorzugsweise eine auf die Feldstärke, das zu fördernde Partikel bzw. Zellverbände enthaltende Material und den gewünschten Effekt abgestimmte optimale und mit hoher Genauigkeit wiederholbare Behandlung des durch den Einlass 7 zugeführten Materials gewährleistet.

Die Vorrichtung 1 zur elektrischen Desintegration kann in einer bevorzugten Ausgestaltung in Anlagen zur Herstellung von Futtermittelzwischenprodukten oder Futtermittelprodukten an verschiedenen Stellen alternativ oder kumulativ vorgesehen werden, um dort zielgerichtet das jeweils geförderte Zellverbände enthaltende Material der elektrischen Desintegration zu unterziehen. Beispielhafte Anlagentypen sind in Figur 2 dargestellt.

In Figur 2 ist schematisch eine Anlage 200 zur Herstellung eines Futtermittelprodukts dargestellt. Die Anlage 200 umfasst auch eine Anlage 100 zur Herstellung eines Futtermittelzwischenprodukts.

Die Anlage 100 weist einen Einlass 101 zum Zuführen von Zellverbände enthaltende Material, beispielsweise Getreideschrot auf. Ferner weist die Anlage 100 einen Auslass 107 zur Abgabe des behandelten Materials als Zwischenprodukt auf. Bestandteil der Anlage 100 ist eine Befeuchtungseinrichtung 103 zur Vorkonditionierung des Materials. Der Befeuchtungseinrichtung 103 wird von dem Einlass 101 kommendes Zellverbände enthaltendes Material zugeführt, und sodann in Richtung des Auslasses 107 wieder abgegeben. Stromabwärts der Befeuchtungseinrichtung 103 ist eine Behandlungseinrichtung 105 vorgesehen, welcher das vorkonditionierte, Zellverbände enthaltende Material zum thermischen und/oder mechanischen Aufschluss zugeführt wird. Vorzugsweise weist die Behandlungseinrichtung einen Extruder und/oder Expander auf.

Die Anlage 100 weist mindestens eine Vorrichtung 1 zur elektrischen Desintegration auf. Die Vorrichtung ist vorzugsweise stromaufwärts oder stromabwärts von der Befeuchtungseinrichtung 103, und/oder stromaufwärts oder stromabwärts der Behandlungseinrichtung 105 angeordnet. Es wird alternativ auch bevorzugt, mehr als eine Vorrichtung 1 zur elektrischen Desintegration in der Anlage 100 vorzusehen. Je nachdem, an welcher Stelle in der Anlage 100 zwischen dem Einlass 101 und dem Auslass 107 die Vorrichtung 1 zur elektrischen Desintegration angeordnet wird, nimmt sie das in seinem jeweiligen Zustand befindliche Zellverbände enthaltende Material auf, unterzieht dies der elektrischen Desintegration und gibt es sodann wieder an den nachfolgenden Bearbeitungsschritt bzw. den Auslass 107 ab (in dem Fall, in dem die Vorrichtung 1 zur elektrischen Desintegration an der in Figur 2 unterstehen Stelle vorgesehen ist).

Die Anlage 100 gemäß Figur 2 ist Bestandteil der Anlage 200 zur Herstellung des Futtermittelprodukts. Im vorliegenden Fall ist die Anlage 200 eine Anlage zur Herstellung eines pelletförmigen Futtermittelprodukts. Zusätzlich zu der Anlage 100 zur Herstellung des Futtermittelzwischenprodukts umfasst die Anlage 200 eine Pelletiereinrichtung 203. Über einen Einlass 201 nimmt die Pelletiereinrichtung 203 das Futtermittelzwischenprodukt auf, welches von der Anlage 100 abgegeben wird. Dies kann unmittelbar geschehen, oder durch Zwischenschaltung geeigneter Transportmittel. Gegebenenfalls findet auch eine Lagerung des Futtermittelzwischenprodukts statt, bevor dieses der Pelletiereinrichtung 203 durch ihren Einlass 201 zugeführt wird. Nach dem Pelletieren gibt die Pelletiereinrichtung 203 durch einen Auslass 205 das fertiggestellte pelletförmige Futtermittelprodukt ab.

Wie vorstehend ausführlich erörtert wurde, stellt die Erfindung in einem Aspekt der Futtermittelindustrie mit der elektrischen Desintegration ein innovatives Hilfsmittel zur Verfügung, mit welchem die Futterverwertung im Verhältnis zu dem für die Herstellung notwendigen Energieaufwand weiter verbessert wird. Vorrichtungen zur elektrischen Desintegration können vorteilhaft sowohl in der Herstellung von Futtermittelzwischenprodukten als auch in der Herstellung von fertigen Futtermittelprodukten verwendet werden.

In einem weiteren Aspekt stellt die Erfindung mit dem im Wesentlichen gleichen apparativen Aufbau ein Werkzeug zur elektrischen Modifikation der Oberflächenladung von Partikel enthaltendem Material zur Verfügung, das die Verarbeitbarkeit des Partikel enthaltenden Materials verbessert.

## Patentansprüche

1. Vorrichtung (1) zur elektrischen Desintegration von Zellverbänden, mit
- einer Kammer (5) mit einem Einlass zur Aufnahme von Zellverbände enthaltendem Material, einem Auslass zu dessen Abgabe, und einer sich zwischen dem Einlass und dem Auslass erstreckenden Förderstrecke (F),
- einer Elektrodeneinheit (11), welche einen Elektrodenkörper (15) aufweist, der innerhalb der Kammer zumindest abschnittsweise entlang der Förderstrecke (F) angeordnet ist, wobei
- die Kammer (5) eine Wand (19) aufweist, die abschnittsweise oder vollständig elektrisch leitfähig und von dem Elektrodenkörper (15) elektrisch isoliert ist, und
- die Elektrodeneinheit (11) dazu angepasst ist, zwischen dem Elektrodenkörper (15) und der Wand (19) ein elektrisches Feld zur elektrischen Desintegration zu erzeugen,
**dadurch gekennzeichnet, dass** in der Förderstrecke (F) eine Förderschnecke (14) zum Fördern des Materials antreibbar angeordnet ist, wobei der Elektrodenkörper (15) in einem Hohlraum der Förderschnecke (14) aufgenommen ist.

2. Vorrichtung nach Anspruch 1,
wobei die Förderschnecke (14) eine Hohlwelle (18) aufweist, und der Elektrodenkörper (15) koaxial zu der Förderschnecke (14) in der Hohlwelle (18) angeordnet ist.

3. Vorrichtung nach Anspruch 1 oder 2,
wobei die Förderschnecke (14) teilweise oder vollständig aus elektrisch nicht leitfähigem Material besteht, wobei vorzugsweise
das elektrisch nicht leitfähige Material ein elektrisch nicht leitfähiges, vorzugsweise organisches, Polymer, oder
ein elektrisch nicht leitfähiger keramischer Werkstoff ist, oder
wobei die Förderschnecke aus zwei oder mehr verschiedenen elektrisch nicht leitfähigen Werkstoffen besteht, vorzugsweise aus einem elektrisch nicht leitfähigen, vorzugsweise organischen, Polymer und einem elektrisch nicht leitfähigen keramischen Werkstoff.

4. Vorrichtung nach einem der vorstehenden Ansprüche,
wobei der Elektrodenkörper (15) in die Hohlwelle (18) eingegossen, vorzugsweise epoxidharzvergossen ist.

5. Vorrichtung nach einem der vorstehenden Ansprüche,
wobei der Elektrodenkörper (15) in der Hohlwelle (18) von einem Isolieröl umgeben ist,
wobei das Isolieröl vorzugsweise ein Mineralöl oder Silikonöl ist.

6. Vorrichtung zur elektrischen Modifikation der Oberflächenladung von Partikeln, mit
- einer Kammer mit einem Materialeinlass, einem Materialauslass, und einer sich zwischen dem Materialeinlass und dem Materialauslass erstreckenden Förderstrecke,
- einer Elektrodeneinheit, welche einen Elektrodenkörper aufweist, der innerhalb der Kammer zumindest abschnittsweise entlang der Förderstrecke angeordnet ist, wobei
- die Kammer eine Wand aufweist, die abschnittsweise oder vollständig elektrisch leitfähig und von dem Elektrodenkörper elektrisch isoliert ist, und
- die Elektrodeneinheit dazu angepasst ist, zwischen dem Elektrodenkörper und der Wand ein elektrisches Feld zur Modifikation der Oberflächenladung zu erzeugen,
**dadurch gekennzeichnet, dass** in der Förderstrecke eine Förderschnecke zum Fördern des Materials antreibbar angeordnet ist, wobei der Elektrodenkörper in einem Hohlraum der Förderschnecke aufgenommen ist.

7. Vorrichtung nach Anspruch 6,
ferner mit den Merkmalen nach einem der vorstehenden Ansprüche 2 bis 5.

8. Anlage (100) zur Herstellung eines Futtermittelzwischenproduktes, mit
- einem Einlass (101) zum Zuführen von Zellverbände enthaltendem Material, und
- einem Auslass (107) zur Abgabe des behandelten Materials als Zwischenprodukt,
**gekennzeichnet durch** eine stromabwärts des Einlasses (101) und stromaufwärts des Auslasses (107) angeordnete, das aus Richtung des Einlasses kommende Material aufnehmende und in Richtung des Auslasses abgebende Vorrichtung (1) zur elektrischen Desintegration von Zellverbänden, die nach einem der Ansprüche 1 bis 5 ausgebildet ist.

9. Anlage (100) nach Anspruch 8,
mit einer Befeuchtungseinrichtung (103) zur Vorkonditionierung, vorzugsweise mittels Sattdampf, des zugeführten Materials, wobei die Vorrichtung zur elektrischen Desintegration stromabwärts oder stromaufwärts der Befeuchtungseinrichtung angeordnet ist; und/oder
mit einer Behandlungseinrichtung (105) zum thermischen und/oder mechanischen Aufschluss des zuggeführten Materials, wobei die Vorrichtung zur elektrischen Desintegration stromaufwärts oder stromabwärts der Behandlungseinrichtung angeordnet ist; und/oder
einer Kühleinrichtung zum Abkühlen des Futtermittelzwischenproduktes.

10. Anlage (200) zur Herstellung eines pelletförmigen Futtermittelproduktes, mit der Anlage (100) zur Herstellung eines Futtermittelzwischenproduktes nach einem der Ansprüche 8 oder 9, und
einer Pelletiereinrichtung (203), die dazu eingerichtet ist, das ihr zugeführte Zwischenprodukt aus der Anlage (100) zur Herstellung des Futtermittelzwischenproduktes aufzunehmen, in Pellets zu verarbeiten, insbesondere zu pressen, und als pelletförmiges Futtermittelprodukt abzugeben.

11. Verwendung einer Vorrichtung (1) zur elektrischen Desintegration in der Herstellung eines Futtermittelzwischenproduktes, wobei die Vorrichtung zur elektrischen Desintegration
- eine Kammer (5) mit einem Einlass (7) zur Aufnahme von Zellverbände enthaltendem Material, einem Auslass (12) zu dessen Abgabe, und einer sich zwischen dem Einlass (7) und dem Auslass (12) erstreckenden Förderstrecke (F), und
- eine Elektrodeneinheit (11) aufweist, welche einen Elektrodenkörper (15) aufweist, der innerhalb der Kammer (5) zumindest abschnittsweise entlang der Förderstrecke (F) angeordnet ist, wobei
- die Kammer (5) eine Wand (19) aufweist, die abschnittsweise oder vollständig elektrisch leitfähig und von dem Elektrodenkörper (15) elektrisch isoliert ist, und
- die Elektrodeneinheit (11) dazu angepasst ist, zwischen dem Elektrodenkörper (15) und der Wand (19) ein elektrisches Feld zur elektrischen Desintegration zu erzeugen,
wobei in der Förderstrecke (F) der Vorrichtung (1) zur elektrischen Desintegration eine Förderschnecke (14) zum Fördern des Materials antreibbar angeordnet ist, wobei der Elektrodenkörper (15) in einem Hohlraum der Förderschnecke (14) aufgenommen ist.

12. Verwendung nach Anspruch 11,
wobei die Vorrichtung (1) zu elektrischen Desintegration nach einem der Ansprüche 2 bis 5 ausgebildet ist.

13. Verwendung einer Vorrichtung (1) zur elektrischen Desintegration in der Herstellung eines Futtermittelproduktes, wobei die Vorrichtung zur elektrischen Desintegration eine Kammer (5) mit einem Einlass (7) zur Aufnahme von Zellverbände enthaltendem Material, einem Auslass (12) zu dessen Abgabe, und einer sich zwischen dem Einlass (7) und dem Auslass (12) erstreckenden Förderstrecke (F), und
- eine Elektrodeneinheit (11) aufweist, welche einen Elektrodenkörper (15) aufweist, der innerhalb der Kammer (5) zumindest abschnittsweise entlang der Förderstrecke (F) angeordnet ist, wobei
- die Kammer (5) eine Wand (19) aufweist, die abschnittsweise oder vollständig elektrisch leitfähig und von dem Elektrodenkörper (15) elektrisch isoliert ist, und
- die Elektrodeneinheit (11) dazu angepasst ist, zwischen dem Elektrodenkörper (15) und der Wand (19) ein elektrisches Feld zur elektrischen Desintegration zu erzeugen,
wobei in der Förderstrecke (F) der Vorrichtung (1) zur elektrischen Desintegration eine Förderschnecke (14) zum Fördern des Materials antreibbar angeordnet ist, wobei der Elektrodenkörper (15) in einem Hohlraum der Förderschnecke (14) aufgenommen ist.

14. Verwendung nach Anspruch 13,
wobei die Vorrichtung (1) zur elektrischen Desintegration nach einem der Ansprüche 2 bis 5 ausgebildet ist.

15. Verwendung einer Vorrichtung zur elektrischen Modifikation der Oberflächenladung von Partikeln, wobei die Vorrichtung nach einem der Ansprüche 6 oder 7 ausgebildet ist.

## Claims

1. A device (1) for the electrical disintegration of cell structures, comprising
- a chamber (5) having an inlet for receiving material containing cell structures, an outlet for the discharge thereof, and a conveyor line (F) extending between the inlet and the outlet,
- an electrode unit (11) which has an electrode body (15) which is arranged within the chamber at least portion-wise along the conveyor line (F), wherein
- the chamber (5) has a wall (19) which is portion-wise or completely electrically conductive and electrically insulated from the electrode body (15), and
- the electrode unit (11) is adapted to generate an electric field between the electrode body (15) and the wall (19) for electrical disintegration,
**characterised in that** a conveyor screw (14) is arranged driveably in the conveyor line (F) for conveying the material, wherein the electrode body (15) is accommodated in a cavity in the conveyor screw (14).

2. A device as set forth in claim 1 wherein the conveyor screw (14) has a hollow shaft (18) and the electrode body (15) is arranged coaxially with the conveyor screw (14) in the hollow shaft (18).

3. A device as set forth in claim 1 or claim 2 wherein the conveyor screw (14) partly or completely comprises electrically non-conductive material, wherein preferably
the electrically non-conductive, preferably organic, polymer is used as the electrically non-conductive material or
is an electrically non-conductive ceramic material or
wherein the conveyor screw comprises two or more different electrically non-conductive materials, preferably an electrically non-conductive, preferably organic, polymer and an electrically non-conductive ceramic material.

4. A device as set forth in one of the preceding claims wherein the electrode body (15) is molded into the hollow shaft (18), preferably epoxy resin molded.

5. A device as set forth in one of the preceding claims wherein the electrode body (15) is surrounded in the hollow shaft (18) by an insulating oil,
wherein the insulating oil is preferably a mineral oil or a silicone oil.

6. A device for the electrical modification of the surface charge of particles, comprising
- a chamber having a material inlet, a material outlet and a conveyor line extending between the material inlet and the material outlet,
- an electrode unit which has an electrode body which is arranged within the chamber at least portion-wise along the conveyor line,
wherein
- the chamber has a wall which is portion-wise or completely electrically conductive and is electrically insulated from the electrode body, and
- the electrode unit is adapted to generate an electric field for modification of the surface charge between the electrode body and the wall,
**characterised in that** a conveyor screw is arranged driveably in the conveyor line for conveying the material, wherein the electrode body is accommodated in a cavity in the conveyor screw.

7. A device as set forth in claim 6 and further comprising the features as set forth in one of preceding claims 2 through 5.

8. Installation (100) for producing a feed intermediate comprising
- an inlet (101) for the feed of material containing cell structures, and
- an outlet (107) for the discharge of the treated material as an intermediate product, **characterised by** a device (1) for the electrical disintegration of cell structures, which is designed as set forth in one of claims 1 through 5, being arranged downstream of the inlet (101) and upstream of the outlet (107) and receiving the material coming from the direction of the inlet and discharging same in the direction of the outlet.

9. Installation (100) as set forth in claim 8 comprising a moistening device (103) for preconditioning, preferably by means of saturated steam, of the supplied material, wherein the device for electrical disintegration is arranged downstream or upstream of the moistening device; and/or
comprising a treatment device (105) for thermal and/or mechanical break-up of the supplied material, wherein the device for electrical disintegration is arranged upstream or downstream of the treatment device; and/or.
comprising a cooling device for cooling down the feed intermediate.

10. Installation (200) for the production of a pellet-form feed product comprising the installation (100) for production of a feed intermediate as set forth in one of claims 8 or 9, and a pelletting device (203) which is adapted to receive the intermediate product supplied thereto from the installation (100) for producing the feed intermediate, to process it as pellets, in particular to press it, and to discharge it as pellet-form feed product.

11. Use of a device (1) for electrical disintegration in the production of a feed intermediate, wherein the device for electrical disintegration comprises
- a chamber (5) having an inlet (7) for receiving material containing cell structures, an outlet (12) for the discharge thereof, and a conveyor line (F) extending between the inlet (7) and the outlet (12),
- an electrode unit (11) which has an electrode body (15) which is arranged within the chamber at least portion-wise along the conveyor line (F), wherein
- the chamber (5) has a wall which is portion-wise or completely electrically conductive and electrically insulated from the electrode body (15), and
- the electrode unit (11) is adapted to generate an electric field between the electrode body (15) and the wall (19) for electrical disintegration,
wherein a conveyor screw (14) for conveying the material is driveably arranged in the conveyor line (F) of the device (1) for electrical disintegration, wherein the electrode body (15) is accommodated in a cavity in the conveyor screw (14).

12. Use as set forth in claim 11 wherein the device (1) for electrical disintegration is preferably as set forth in one of claims 2 through 5.

13. Use of a device (1) for electrical disintegration in the production of a feed product, wherein the device for electrical disintegration has a chamber (5) having an inlet (7) for receiving material containing cell structures, an outlet (12) for the discharge thereof, and a conveyor line (F) extending between the inlet (7) and the outlet (12), and
- an electrode unit (11) having an electrode body (15) which is arranged within the chamber (5) at least portion-wise along the conveyor line (F), wherein
- the chamber (5) has a wall (19) which is portion-wise or completely electrically conductive and is electrically insulated from the electrode body (15), and
- the electrode unit (11) is adapted to generate an electric field between the electrode body (15) and the wall (19) for electrical disintegration,
wherein a conveyor screw (14) for conveying the material is driveably arranged in the conveyor line (F) of the device (1) for electrical disintegration, wherein the electrode body (15) is accommodated in a cavity in the conveyor screw (14).

14. Use as set forth in claim 13 wherein the device (1) for electrical disintegration is as set forth in one of claims 2 through 5.

15. Use of a device for the electrical modification of the surface charge of particles, wherein the device is as set forth in one of claims 6 and 7.

## Revendications

1. Dispositif (1) de désintégration électrique d'amas cellulaires, avec
- une chambre (5) avec une entrée servant à recevoir de la matière contenant des amas cellulaires, une sortie servant à la distribution de celle-ci, et une ligne de transport (F) s'étendant entre l'entrée et la sortie,
- une unité d'électrodes (11), laquelle présente un corps d'électrode (15), qui est disposé à l'intérieur de la chambre au moins par endroits le long de la ligne de transport (F), dans lequel
- la chambre (5) présente une paroi (19), qui est par endroits ou en totalité électriquement conductrice et est isolée électriquement du corps d'électrode (15), et
- l'unité d'électrodes (11) est adaptée pour générer un champ électrique servant à la désintégration électrique entre le corps d'électrode (15) et la paroi (19),
**caractérisé en ce qu'**une vis sans fin de transport (14) servant à transporter la matière est disposée de manière à pouvoir être entraînée sur la ligne de transport (F), dans lequel le corps d'électrode (15) est reçu dans un espace creux de la vis sans fin de transport (14).

2. Dispositif selon la revendication 1,
dans lequel la vis sans fin de transport (14) présente un arbre creux (18), et le corps d'électrode (15) est disposé de manière coaxiale par rapport à la vis sans fin de transport (14) dans l'arbre creux (18).

3. Dispositif selon la revendication 1 ou 2,
dans lequel la vis sans fin de transport (14) est constituée en partie ou en totalité d'une matière non conductrice électriquement, dans lequel de préférence
la matière non conductrice électriquement est un polymère non conducteur électriquement, de préférence organique, ou
un matériau céramique non conducteur électriquement, ou
dans lequel la vis sans fin de transport est constituée de deux matériaux différents non conducteurs électriquement ou plus, de préférence d'un polymère non conducteur électriquement, de préférence organique et d'un matériau céramique non conducteur électriquement.

4. Dispositif selon l'une quelconque des revendications précédentes,
dans lequel le corps d'électrode (15) est coulé dans l'arbre creux (18), en particulier est scellé par coulée dans de la résine époxy.

5. Dispositif selon l'une quelconque des revendications précédentes,
dans lequel le corps d'électrode (15) est entouré dans l'arbre creux (18) d'une huile isolante,
dans lequel l'huile isolante est de préférence une huile minérale ou une huile de silicone.

6. Dispositif de modification électrique de la charge de surface de particules, avec
- une chambre avec une entrée de matière, une sortie de matière et une ligne de transport s'étendant entre l'entrée de matière et la sortie de matière,
- une unité d'électrodes, laquelle présente un corps d'électrode, qui est disposé à l'intérieur de la chambre au moins par endroits le long de la ligne de transport, dans lequel
- la chambre présente une paroi, qui est par endroits ou en totalité électriquement conductrice et est isolée électriquement du corps d'électrode, et
- l'unité d'électrodes est adaptée pour générer un champ électrique servant à la modification de la charge de surface entre le corps d'électrode et la paroi
**caractérisé en ce qu'**une vis sans fin de transport servant à transporter la matière est disposée de manière à pouvoir être entraînée dans la ligne de transport, dans lequel le corps d'électrode est logé dans un espace creux de la vis sans fin de transport.

7. Dispositif selon la revendication 6,
en outre avec les caractéristiques selon l'une quelconque des revendications précédentes 2 à 5.

8. Installation (100) servant à fabriquer un produit intermédiaire alimentaire pour animaux, avec
- une entrée (101) servant à amener de la matière contenant des amas cellulaires, et
- une sortie (107) servant à distribuer la matière traitée en tant que produit intermédiaire,
**caractérisée par** un dispositif (1) disposé en aval de l'entrée (101) et en amont de la sortie (107), recevant la matière provenant de l'entrée et la distribuant en direction de la sortie, servant à la désintégration électrique d'amas cellulaires, qui est réalisé selon l'une quelconque des revendications 1 à 5.

9. Installation (100) selon la revendication 8,
avec un système d'humidification (103) servant au préconditionnement, de préférence au moyen de vapeur saturée, de la matière amenée, dans laquelle le dispositif de désintégration électrique est disposé en aval ou en amont du système d'humidification ; et/ou
avec un système de traitement (105) servant à la désagrégation thermique et/ou mécanique de la matière amenée, dans laquelle le dispositif de désintégration électrique est disposé en amont ou en aval du système de traitement ; et/ou
un système de refroidissement servant à refroidir le produit intermédiaire alimentaire pour animaux.

10. Installation (200) servant à fabriquer un produit alimentaire pour animaux sous forme de granulés, avec l'installation (100) servant à fabriquer un produit intermédiaire alimentaire pour animaux selon l'une quelconque des revendications 8 ou 9, et
un système de granulation (203), qui est conçu pour recevoir le produit intermédiaire qui lui est amené, provenant de l'installation (100) servant à fabriquer le produit intermédiaire alimentaire pour animaux, le transformer, en particulier le presser, en granulés et le distribuer en tant que produit alimentaire pour animaux sous forme de granulés.

11. Utilisation d'un dispositif (1) de désintégration électrique lors de la fabrication d'un produit intermédiaire alimentaire pour animaux, dans laquelle le dispositif de désintégration électrique présente,
- une chambre (5) avec une entrée (7) servant à recevoir de la matière contenant des amas cellulaires, une sortie (12) servant à la distribution de celle-ci et une ligne de transport (F) s'étendant entre l'entrée (7) et la sortie (12), et
- une unité d'électrodes (11), laquelle présente un corps d'électrode (15), qui est disposé à l'intérieur de la chambre (5) au moins par endroits le long de la ligne de transport (F), dans laquelle
- la chambre (5) présente une paroi (19), qui est par endroits ou en totalité électriquement conductrice et est isolée électriquement du corps d'électrode (15), et
- l'unité d'électrodes (11) est adaptée pour générer un champ électrique servant à la désintégration électrique entre le corps d'électrode (15) et la paroi (19)
dans laquelle une vis sans fin de transport (14) servant à transporter la matière est disposée de manière à pouvoir être entraînée dans la ligne de transport (F) du dispositif (1) de désintégration électrique, dans laquelle le corps d'électrode (15) est reçu dans un espace creux de la vis sans fin de transport (14).

12. Utilisation selon la revendication 11,
dans laquelle le dispositif (1) de désintégration électrique est réalisé selon l'une quelconque des revendications 2 à 5.

13. Utilisation d'un dispositif (1) de désintégration électrique lors de la fabrication d'un produit alimentaire pour animaux, dans laquelle le dispositif de désintégration électrique présente une chambre (5) avec une entrée (7) servant à recevoir de la matière contenant des amas cellulaires, une sortie (12) servant à la distribution de celle-ci, et une ligne de transport (F) s'étendant entre l'entrée (7) et la sortie (12), et
- une unité d'électrodes (11), laquelle présente un corps d'électrode (15), qui est disposé à l'intérieur de la chambre (5) au moins par endroits le long de la ligne de transport (F), dans laquelle
- la chambre (5) présente une paroi (19), qui est par endroits ou en totalité électriquement conductrice et est isolée électriquement du corps d'électrode (15), et
- l'unité d'électrodes (11) est adaptée pour générer un champ électrique servant à la désintégration électrique entre le corps d'électrode (15) et la paroi (19)
dans laquelle une vis sans fin de transport (14) servant à transporter la matière est disposée de manière à pouvoir être entraînée sur la ligne de transport (F) du dispositif (1) de désintégration électrique, dans laquelle le corps d'électrode (15) est reçu dans un espace creux de la vis sans fin de transport (14).

14. Utilisation selon la revendication 13,
dans laquelle le dispositif (1) servant à la désintégration électrique est réalisé selon l'une quelconque des revendications 2 à 5.

15. Utilisation d'un dispositif de modification électrique de la charge de surface de particules, dans laquelle le dispositif est réalisé selon l'une quelconque des revendications 6 ou 7.
